(19) Europäisches Patentamt
European Patent Office
Office européen des brevets

(11) **EP 2 351 554 B1**

(12) **EUROPEAN PATENT SPECIFICATION**

(45) Date of publication and mention
of the grant of the patent:
**09.09.2015 Bulletin 2015/37**

(51) Int Cl.:
**A61K 9/00** *(2006.01)* **A61K 31/137** *(2006.01)*

(21) Application number: **11163553.8**

(22) Date of filing: **11.07.2007**

(54) **Enhanced stability phenylephrine liquid compositions**

Flüssige Zubereitungen mit erhöhter Stabilität enthaltend Phenylephrin

Compositions liquides de phényléphrine à stabilité améliorée

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **14.07.2006 US 487120**

(43) Date of publication of application:
**03.08.2011 Bulletin 2011/31**

(62) Document number(s) of the earlier application(s) in accordance with Art. 76 EPC:
**07836046.8 / 2 040 672**

(73) Proprietor: **Wyeth LLC**
**New York, NY 10017-5755 (US)**

(72) Inventors:
• **Bubnis, William**
**Mechanicsville, VA 23116 (US)**
• **Shield, Stephanie**
**Richmond, VA 23235 (US)**

• **Hoskovec, Gayle, P.**
**Quinton, VA 23141 (US)**

(74) Representative: **Pfizer**
**European Patent Department**
**23-25 avenue du Docteur Lannelongue**
**75668 Paris Cedex 14 (FR)**

(56) References cited:
**WO-A-96/23486** **WO-A-03/047502**
**WO-A-2005/056635** **WO-A-2007/125501**
**US-A1- 2003 194 441**

• **LI ET AL: "Detection and quantification of low-molecular-weight aldehydes in pharmaceutical excipients by headspace gas chromatography", JOURNAL OF CHROMATOGRAPHY A, ELSEVIER, AMSTERDAM, NL, vol. 1104, no. 1-2, 3 February 2006 (2006-02-03), pages 1-10, XP24968259, ISSN: 0021-9673**

## Description

An oral liquid pharmaceutical composition comprising phenylephrine is provided. The composition is particularly well suited for the of cold, cough, flu, fever, headache, pain, body ache, migraine, and allergy symptoms.

## BACKGROUND OF THE INVENTION

Orally administered pharmaceutical compositions are provided to patients in many dosage forms, Including solid forms such as capsules, caplets or tablets and liquid forms such as solution and suspensions, For many patients including young children, older persons and incapacitated persons, a liquid dose form is preferable because of the ease with which it may be swallowed.

Many commercially available over-me-counter liquid cold, cough, flu, fever, and/or allergy preparations contain pseudoephedrine as an active agent Although such preparations have been useful, misuse of such products as a starting material for synthesis of illicit substance has lead to the desire to find alternative that are not suitable for such illicit synthesis. Phenylephrine is a potential alternative active. However, phenylephrine is susceptible to degradation. The degradation is typically facilitated in excipient compositions of the type typically used with pseudoephedrine.

Accordingly, it would be desirable to have a palatable, liquid dosage form comprising Phenylephrine with reduced propensity for degradation of phenylephrine.

## SUMMARY OF THE INVENTION

The invention provides an oral liquid pharmaceutical composition comprising:

(a) phenylephrine or a pharmaceutically acceptable salt thereof;

(b) substantially aldehyde-free polyethylene glycol, wherein the substantially aldehyde-free polyethylene glycol has less than 10 ppm total aldehyde content and maintains said level of aldehyde content for at least one year;

(c) a second active agent selected from an analgesic, a decongestant, an expectorant, an anti-tussive, an antipyretic, an anti-inflammatory agent, a cough suppressant and an antihistamine; and

(d) a buffering agent which maintains a pH below 5.4 in the composition.

The composition may be a solution or a suspension. Suspension embodiments may further comprise viscosity modifying agents. In some embodiments the composition may be filled into capsules.

## BRIEF DESCRIPTION OF THE FIGURES

FIGURE 1

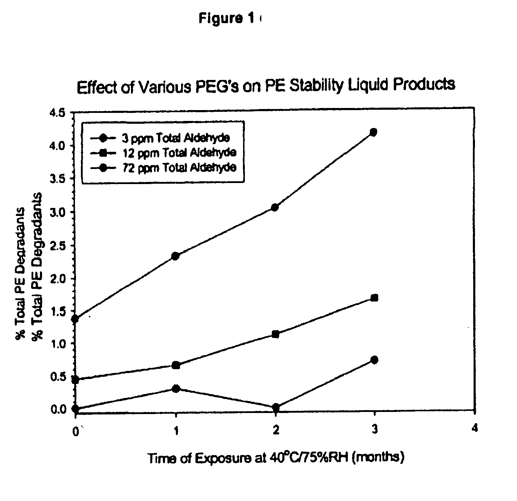

Figure 1 is a plot showing degradation of phenylephrine in a polyethylene glycol composition as a function of total aldehyde concentration in the polyethylene glycol.

## DETAILED DESCRIPTION OF THE INVENTION

The composition is palatable and has improved phenylephrine stability. The composition of the invention may be a solution or a suspension or alternatively filled into capsules. In solution and suspension embodiments, the composition comprises phenylephrine, an artificial sweetener, and substantially aldehyde-free polyethylene glycol.

Applicants believe without wishing to be held to the theory that Phenylephrine degradation is facilitated by the presence of aldehydes and reducing sugars (see Applicants co-pending provisional application 60/774,634). Applicants have made the discovery that many "high purity" polyethylene glycols (e.g. "PEG"), have a significant amount of aldehyde impurity (e.g. impurities and/or degradant components that bear an aldehyde functionality) upon receipt from commercial suppliers whether this is an artifact of the production process, the result of oxidation of the raw materials, or the result of another cause is unknown. Applicants further discovered that if this PEG with significant aldehyde content is used in a phenylephrine composition, degradation of the phenylephrine is facilitated yielding a product of dubious, if not inadequate, stability for a commercial product.

Accordingly applicants have discovered that phenylephrine stability compatible with commercial product requirements can be obtained by using substantially aldehyde-free polyethylene glycol. As used herein, "substantially aldehyde-free polyethylene glycol" (i.e. "SAF-PEG") means a polyethylene glycol with less than 20 ppm total aldehyde content, and preferably less than 10 ppm total aldehyde content and that the polyethylene glycol can maintain a total aldehyde content below 20 ppm and preferably below 10 ppm for at least six months and preferably for at least one year.

The total aldehyde content in the SAF-PEG may be measured using an HPLC method that provides for identification and quantification of each component bearing an aldehyde functionality. The total aldehyde content is the sum of the individual aldehyde components. Note that the SAF-PEG definition refers to the composition of the polyethylene glycol as a single entity prior to use of the SAF-PEG to form the composition of the invention (e.g. it is a specification of the polyethylene glycol and not of the final composition). Stability of the SAF-PEG to maintain the designated aldehyde levels over time may be determined by maintaining aliquots of SAF-PEG in closed containers, preferably, with minimal contact to light and repeating the HPLC testing for total aldehyde concentration on the maintained aliquots after a predetermined amount of time.

SAF-PEG may be obtained commercially from Sasol Germany GmbH, Werk Marl, Paul-Baumann-Str., Germany. Alternatively, SAF-PEG may be obtained by purification of commercial PEG with higher levels of aldehyde, e.g. removal of aldehydes or reduction of aldehyde content to the specified parameters,

Preferably the phenylephrine is in a salt form. Suitable salt forms include, but are not limited to, phenylephrine hydrochloride (HCl), hydrobromide (HBr), bitartarate and tannate salts. Phenylephrine may be used In an amount of about 0,001% w/v to about 10% w/v.

Preferably, phenylephrine is used in an amount of about 0.005% w/v to about 2.5%w/v. Herein % w/v means a percentage determined by the following formula:

$$w/v\ \% = \underline{\frac{\text{Weight of component (in grams)}}{\phantom{xxxxxxxxxxxxxxxxxxxxxxxxxxxxxxxxxxxxxxx}}} \qquad X \qquad 100$$

$$\text{Volume of composition (in milliliters)}$$

Accordingly, for example, 1% w/v% Phenylephrine means 1 gram of phenylephrine in 100 ml of the oral liquid composition.

An artificial sweetener may be provided to improve palatability, An artificial sweetener is preferred for use as a sweetener to the use of conventional sugar sweeteners as the inventors believe, without wishing to be held to the theory, that conventional sugars may contribute to the degradation of phenylephrine in aqueous based compositions. Suitable artificial sweeteners, include but are not limited to sucralose, saccharine salts, cyclamates, acesulfame K, dipeptide based sweeteners, aspartame and mixtures thereof. Sucralose, which is a high intensity sweetener, is particularly well suited for use in the composition. Sucralose may be used in an amount of about 0.01% w/v to about 0.4% w/v, for example. The appropriate amount of artificial sweetener depends on properties and sweetness intensity of the artificial sweetener and target organoleptic properties of the composition. One skilled in the art is familiar with the characteristics of sweeteners and methods for determining amount of sweetener to be used.

Optionally, glycerin and sorbitol may be used in solution and suspension embodiments of the composition. Like many conventional commercial cold products, in one embodiment the composition may comprise more sorbitol than glycerin. Alternatively, in one embodiment the composition contains more glycerin than sorbitol. The inventors believe, without wishing to be bound to the theory, that reduced amounts of sorbitol facilitate stability of the phenylephrine. The composition may contain up to 45% w/v glycerin and up to about 50% w/v sorbitol. In exemplary embodiments with reduced sorbitol amounts, the composition may contain about 18% to about 30% w/v glycerin and about 3% to about 10% w/v sorbitol. Herein the amounts of sorbitol and glycerin are the amounts of standard commercial preparations of sorbitol and glycerin. Commercial sorbitol (as obtained from SPI Polyols, 321 Cherry Lane New Castle, Delaware 19720, or Roquette Frèves 62080 Lestrew, France, for example) is an aqueous based composition that is 70% sorbitol. Commercial glycerin (as obtained from Dow Chemical Co., 2030 Dow Center, Midland, MI 48674, or Lyondell, 1221 McKinney St., Houston, TX 77253, for example) is 96% glycerin. One skilled in the art is familiar with these commercial preparations and methods of adjusting amounts should a different glycerin preparation (such as, for example, a 99% glycerin) or a different sorbitol preparation be used.

The composition contains one or more additional pharmaceutical actives (also referred to as "active(s)", "active agent(s)", "therapeutic agent(s)", "drug(s)"). Herein reference to "first pharmaceutical active" means phenylephrine and reference to "second pharmaceutical active" means any active other than phenylephrine. Further, the term second pharmaceutical active may refer to a single species of active or a plurality of species of actives other than phenylephrine (e.g., the total number of actives in the compositions may be greater than 2). For embodiments of the composition that are solutions, any additional active should be water soluble. A water-soluble pharmaceutical active means a pharmaceutical active indicated to be soluble in water by the Merck Index. Additional actives in suspension embodiments may be water soluble, slightly soluble in water, or insoluble in an aqueous medium.

Antihistamines useful in the practice of the present invention (along with their preferred salt form) include, but are not limited to, chlorpheniramine (maleate), brompheniramine (maleate); dexchlorpheniramine (maleate), dexbrompheniramine (maleate), triprolidine (HCl), diphenhydramine (HCl, citrate), doxylamine (succinate), tripelenamine (HCl), cyproheptatine (HCl), chlorcyclizine (HC1), bromodiphenhydramine (HCl), phenindamine (tartrate), pyrilamine (maleate, tannate), azatadine (maleate); acrivastine, astemizole, azelastine, cetirizine, ebastine, fexofenadine, ketotifen, carbinoxamine (maleate), desloratadine, loratadine, pheniramine maleate, thonzylamine (HCl), mizolastine and terfenadine.

Antitussives useful in the practice of the present invention (along with their preferred salt form) include, but are not limited to, chlophendianol, caramiphen (ediylate), dextromethorphan (HBr), diphenhydramine (citrate, HCl), codeine (phosphate, sulfate) and hydrocodone.

Decongestants useful in the practice of the invention (along with their preferred salt form) include, but are not limited to, pseudoephedrine (HCl, sulfate), ephedrine (HCl, sulfate), phenylephrine (bitartarate, tannate, HBr, HCl), and phenylpropenolamine (HCl).

Expectorants which may be used in the practice of the invention (along with their preferred salt form) include but are not limited to terpin hydrate, guaifenesin (glycerol, guaiacolate), potassium (iodide, citrate) and potassium guaicolsulfonate.

Non-steroidal anti-inflammatory drugs (NSAIDS) which may be used in the practice of the invention include, but are not limited to, propionic acid derivatives such as ibuprofen, naproxen, ketoprofen, flurbiprofen, fenoprofen, suprofen, fluprofen and fenbufen; acetic acid derivatives such as tolmetin sodium, zomepirac, sulindac, and indomethacin; fenamic acid derivatives such as mefenamic acid and meclofenamate sodium; biphenyl carboxylic acid derivatives such as diflunisal and flufenisal and oxicams such as piroxicam, sudoxicam and isoxicam.

Cox 2 inhibitors which may be used in the practice of the invention include, but are not limited to, Celecoxib, Rofecoxib and Valdecoxib.

Analgesics which may be used in the practice of the invention include but are not limited to aspirin, acetominophen, phenacetin and salicylate salts.

Examples of substantially insoluble pharmaceutical actives that may be suspended in the suspending system of suspension embodiments include, but are not limited to, nabumetone, glimepiride, diclofenac, piroxicam and meloxican.

Of the pharmaceutically active compounds described above which may be included in addition to phenylepherine in the composition, those which are particularly preferred are set forth below along with preferred ranges for their inclusion into the claimed pharmaceutical composition.

Chlorpheniramine may be used in the pharmaceutical composition in amounts between about 0.01% w/v and about 0.05% w/v, Preferably chlorpheniramine, when used in the pharmaceutical composition, is present in the amount of about 0,01% w/v to 0.03% w/v.

Chlorpheniramine maleate may be used in the pharmaceutical composition, preferably in the amount of about 0.01% w/v to about 0.03% w/v.

Brompheniramine maleate may be used in the pharmaceutical composition, Preferably in the amount of about 0,01% w/v to about 0.03% w/v.

Dextromethorphan HBr may be used in the pharmaceutical composition, preferably In the amount of about 0.05% w/v to about 0.250% w/v.

Guaifenesin may be used in the composition in amounts of about 0.4 % w/v to about 6 % w/v and preferably in amounts of about 2 % w/v to about 4 % w/v.

Acetaminophen may be used in the composition in amounts of about 0.2 % w/v to about 10 % w/v and in amounts of about 0.5 % w/v to about 3.2 % w/v.

Chlophendianol may be used in the composition in amounts of about 0.1 % w/v to about 1 % w/v and preferably in amounts of about 0.25 % w/v to about 0.5 % w/v.

Diphenhydramine may be used in the composition in amounts of about 0.2 % w/v to about 2 % w/v and preferably in amounts of about 0.5 % w/v to about 1 % w/v.

Brompheniramine may be used in the composition in amounts of about 0.016 % w/v to about 0.16% w/v and preferably in amounts of about 0.02% w/v to about 0.08 % w/v.

Loratadine may be used in the composition in amounts of about 0.02 % w/v to about 0.4 % w/v and preferably in amounts of about 0.1 % w/v to about 0.2 % w/v.

Aspirin may be used in the composition in amounts of about 0.8 % w/v to about 13 % w/v and preferably in amounts of about 3.2 % w/v to about 7.2 % w/v.

Doxylamine may be used in the composition in amounts of about 0.1% w/v to about 1% w/v and preferably in amounts about 0.25 % w/v to about 0.5 % w/v.

Acetaminophen may be used in the composition in amounts of about 0.12 % w/v to about 13 % w/v and preferably in amounts of about 1.2%w/v to about 4 %w/v.

Amounts of pharmaceutically active compounds incorporated are conventional dosages known to those skilled in the art. Further, for pharmaceutical compositions intended for use in the United States, amounts of pharmaceutical actives are preferably in compliance with applicable FDA regulations regarding dosage of such compounds.

The pharmaceutically active compounds are preferably, but not limited to, a compendial grade such as, for example, N.F, (National Formulary) or U.S.P. (United States Pharmacopeia) grade.

Excipients known by those skilled in the art may be useful in the practice of the present invention. Such excipients may include, but are not limited to, humectants such as glycerin, sweeteners, defoaming agents, buffers, electrolytes, preservatives such as sodium benzoate and disodium edetate, antioxidants, taste masking agents and various flavoring and coloring agents, for example. Optionally, some embodiments may include viscosity modifiers such as, for example, glycerin, xanthan, and /or povidone; and/or densifiers such as, for example, sorbitol or glycerin.

Examples of suitable flavoring agents include, but are not limited to, natural and artificial flavors such as mints (i.e., peppermint, etc.), menthol, chocolate, artificial chocolate, bubblegum, both artificial and natural fruit flavors (i.e., cherry, grape, orange, strawberry, etc.) and combinations of two or more thereof. It is preferable to avoid flavoring agents which have aldehyde functional groups (e.g. use non-aldehyde containing flavorants is preferred). Flavoring agents are generally provided as a minor component of the composition in amounts effective to provide palatable flavor to the compositions. Typically, flavoring agents are present in amounts in the range of about 0 % wt/v to about 5 % wt/v in the composition.

Optionally, an antioxidant may be used in the composition. Propyl gallate is exemplary of an antioxidant that is suitable for use in the composition.

Preservatives useful in the present invention include but are not limited to sodium benzoate, sorbates, such as potassium sorbate, salts of edetate (also known as salts of ethylenediaminetetraacetic acid or EDTA, such as disodium edetate), benzaldionium chloride and parabens (such as methyl, ethyl, propyl, and butyl p-hydroxybenzoic acid esters). Preservatives listed above are exemplary, but each preservative must be evaluated on an experimental basis, in each formulation to assure compatibility and efficacy of the preservative. Methods for evaluating the efficacy of preservatives in pharmaceutical formulations are known to those skilled in the art. Sodium benzoate and disodium edetate are the presently preferred preservative ingredients.

Preservatives are generally present in amounts of up to one gram per 100 ml of the pharmaceutical composition. Preferably the preservatives are present in amounts in the range of from about 0.01 % w/v to about 0.4 % w/v of the composition. Typically, the preservative sodium benzoate would be present in the range of about 0.1 % w/v to about 0.2% w/v of the composition, for example. Sodium benzoate was used in a concentration of about 0.1% w/v in an exemplary embodiment of the composition.

Sodium citrate is exemplary of a buffering agent which may be used in the composition. Preferably the pH may be maintained in the range of about pH 2 to about pH 5. Coloring agents may also be incorporated in the pharmaceutical composition to provide an appealing color to the composition. The coloring agents should be selected to avoid chemical incompatibilities with other ingredients in the composition. Suitable coloring agents are well known to those skilled in the art.

In some embodiments, particularly suspension embodiments, a surface modifying agent, such as a surfactant, may be used in the pharmaceutical composition to modify the surface of the suspended components. Such surface modification is believed to facilitate diminished irreversible aggregation of the suspended particles. The surfactant may be an ionic or non-ionic surfactant or mixtures thereof. Exemplary surfactants include but are not limited to polysorbates (tweens), Spans™, togats, lecithin, polyoxyethylene-polyoxypropylene block copolymers and medium chain mono/di-glycerides.

Typically, suspension embodiments will further comprise a viscosity modifying agent. Suitable viscosity modifying agents include but are not limited to chitosan, xanthan, povidone, hydroxpropylmethylcellulose (HPMC), hydroxypropylcellulose (HPC), hydroxyethylcellulose (HEC), galactomannans such as guar, konjac, locust bean gum and mamman, for example, microcrystalline cellulose and combinations thereof.

Xanthan gums suitable for use in the present invention are high molecular weight polysaccharides such as the xanthan gum produced by *Xanthamonas capestris,* for example. Xanthan gum is an article of commerce and is available, for example, from manufacturers such as: Rhodia, Inc. under the brand name Rhodigel™ and from Kelco™, a division of Merck. Rhodigel™ 80 Pharm Grade is exemplary of one specific commercial product suitable for use in the practice of the invention.

Microcrystalline cellulose is commercially available from suppliers such as FMC (1735 Market Street, Philadelphia, PA 19103) under the tradename Avicel™.

The amount of viscosity modifier used depends on the desired "thickness" of the composition and the type viscosity modifier used. Combinations of viscosity modifiers may be employed. For example, in an exemplary embodiment with a viscosity of about 1500 to about 4500 cps, up to about 1,0% w/v xanthan gum may be used with up to about 3,0% w/v microcrystalline cellulose may be as a viscosity modifier.

It is preferable to avoid viscosity modifiers with a significant presence of negatively charged moieties or moieties with propensity to ionize to a negative charge if the structure of the modifier is such that the negatively charged moiety is readily available for reaction.

Suspensions are useful for preparing compositions comprising actives that are substantially insoluble in water. In suspension embodiments the phenylephrine is dissolved in the aqueous medium. The composition may contain one or more second active agents dissolved in the aqueous medium and/or one or more substantially water Insoluble second active agents may be suspended in the composition. For the suspension embodiments, it is preferable that both the suspended Substantially Insoluble active ingredients and any soluble active ingredients dissolved in the aqueous medium, are distributed to form a substantially homogeneous distribution of active ingredients in the pharmaceutical composition.

Exemplary pharmaceutical actives that are substantially Insoluble in the aqueous composition and would be expected to form suspension include but are not limited to Ibuprofen, Ketoprofen, Naproxen, Celecoxib, Rofecoxib, Valdecoxib, Nabumetone, Glimepiride, Diclofenac, Piroxicam and Meloxican. For pharmaceutical actives not specified on this list a pharmaceutical active substantially insoluble in the aqueous composition means a pharmaceutical active designated as relatively insoluble or insoluble in water by the Merck Index.

Typically, solution and suspension forms of the composition are provided to a patient in need of treatment in a dosage unit of 5 ml Although other dosage units may be likewise suitable. The dosage unit may be provided as a single dosage unit or multiples thereof, based on age, weight and other health parameters determined by a physician to be relevant.

Alternatively, the composition may be prepared as a liquid fill for capsules. In an exemplary liquid fill embodiment, the composition comprises SAF-PEG and phenylephrine. Optionally, at least one second active agent may be included in the composition. In one exemplary embodiment comprising a second active agent, the composition comprises SAF-PEG, phenylephrine, ibuprofen and an aqueous alkali solution such as 50% potassium hydroxide, for example. The composition may be filled into soft or hard capsules.

EXAMPLE 1 (Illustrative)

A composition comprising the single first pharmaceutical active phenylephrine is provided in Table 1. The embodiment is provided for illustrative purposes.

Table 1

| Ingredient | Amount (grams/100ml x 100) |
|---|---|
| Phenylephrine HCl | 0.1 % w/v |
| Glycerin (96% USP) | 25% w/v |
| Sorbitol (70% Solution USP) | 10% w/v |
| Micronized Sucralose Powder (NF) | 0.2% w/v |
| Substantially aldehyde-free polyethylene glycol (commercial, <10ppm) | 10.0% w/v |
| colorant | 0.01 % w/v |
| sodium citrate/citric acid | 0.95% w/v |
| sodium benzoate | 0.1% w/v |
| purified $H_2O$ USP | sufficient quantity to make final volume |

The composition of Table 1 is prepared by simple mixing. The ingredients are mixed in a vessel equipped with a mechanical stirrer (e.g., a Lightnin mixer), the vessel is calibrated and marked to designate the final volume. An aliquot of water substantially less than the target final volume is placed in the vessel and the SAF-PEG is added and mixed with the water. The phenylephrine is added to the solution in the vessel with mixing. The other ingredients are added sequentially with mixing. Colorants may be added directly or premixed with a small amount of water prior to addition to the main vessel. After all other ingredients are added and mixed sufficiently to dissolve, water is added to bring the total volume of the composition to the predetermined final volume and mixing is continued for approximately 10 minutes.

EXAMPLE 2

An exemplary composition comprising phenylephrine and a second active dextromethorphan hydrobromide is provided in Table 2. This composition is representative and one of the many compositions that are within the scope of

EP 2 351 554 B1

the invention. The exemplary embodiments is provided for illustrative purposes.

TABLE 2

| Ingredient | Amount (grams/100 ml + 100) |
|---|---|
| Phenylephrine HCl | 0.1% w/v |
| Dextromethorphan Hydrobromide | 0.02% w/v |
| Glycerin (96% USP) | 25% w/v |
| Sorbitol (70% Solution USP) | 10% w/v |
| Micronized Sucralose | 0.2% w/v |
| Artificial Fruit Flavor | 0 2% w/v |
| Colorant | < 0.1% w/v |
| Sodium Citrate/Citric Acid | 0,95% w/v |
| Sodium Benzoate | 0.1% w/v |
| Substantial aldehyde-free polyethylene glycol (commercial, <10ppm) | 10% w/v |
| Purified $H_2O$ | Sufficient quantity to make final volume |

The composition of Table 2 may be prepared using the manner of preparation described in Example 1. The active agents phenylephrine and dextromethorphan are added to the water SAF-PEG solution prior to the addition of the other excipients,

EXAMPLE 3

An exemplary composition comprising phenylephrine and the two second active agents, dextromethorphan and guaifenesin is provided in Table 3. This composition is representative and one of many composition that are within the scope of the invention. The exemplary embodiment Is provided for illustrative purposes.

TABLE 3

| Ingredient | Amount (grams/100ml x 100) |
|---|---|
| Phenylephrine HCl | 0.1% w/v |
| Dextromethorphan Hydrobromide | 0.2% w/v |
| Guaifenesin | 4% w/v |
| Glycerin (96% USP) | 25% w/v |
| (70% Solution USP) | 10% w/v |
| Micronized Sucralose Powder (NF) | 0.2% w/v |
| colorant | 0.01 % w/v |
| sodium Citrate/citric acid | 0.95% w/v |
| sodium benzoate | 0.1% w/v |
| Substantially aldehyde-free polyethylene glycol (commercial, <10ppm) | 10% w/v |
| purified $H_2O$ USP | sufficient quantity to make final volume |

The composition of Table 3 may be prepared using the manner of preparation described in Example 2.

7

EXAMPLE 4

An exemplary composition comprising and the three second active agents acotaminophen, chlorphoniramine maleate and doxtromothorphan hydrobromide is provided in Table 4. This composition is representative and one of the many compositions that are within the scope of the invention. The exemplary embodiment is provided for illustrative purposes.

TABLE 4

| Ingredient | Amount (grams/100 ml + 100) |
|---|---|
| Phenylephrine HCl | 0.05% w/v |
| Acetaminophen | 3.2% w/v |
| Chlorpheniramine Maleate | 0,02% w/v |
| Destromethorphen Hydrobromide | 0.1 % w/v |
| Sorbitol (70% Solution USP) | 10% w/v |
| Glycerin (96% USP) | 25% w/v |
| Micronized Sucralose | 0,2% w/v |
| Artificial Fruit Flavor | 0.2% w/v |
| Colorant | <0,1% w/v |
| Sodium Citrate/Citric Acid | 0.6% w/v |
| Sodium Benzoate | 0,1% w/v |
| Substantially aldehyde-free polyethylene glycol (commercial, <10ppm) | 20% w/v |
| ProDyl Gallate | 0.1% w/v |
| Purified $H_2O$ | Sufficient quantity to make final volume |

The composition of Table 4 may be prepared using the manner of preparation described in Example 2. Preferably the acetaminophen is added to the water SAF-PEG solution with mixing prior to the addition of the other actives.

Although the foregoing invention has been described in some detail by way of illustrations and examples for purposes of clarity of understanding. It will be obvious that certain changes and modifications may be practiced within the scope of the appended claims. Modifications of the above-described modes of practicing the invention that are obvious to persons of skill in the art are intended to be includes within the scope of the following claims.

EXAMPLE 5

Three compositions comprising 0.1% wt/v phenylephrine in polyethylene glycol were prepared. The compositions were alike in every respect except the polyethylene glycol used. The three polyethylene glycols used contained 3 ppm, 12 ppm and 72 ppm total aldehyde content, respectively. Three month stability testing at conditions of 40 degrees C and 75% relatively humidity was conducted on the three samples. As Figure 1 shows, at the end of the three month test period the sample made from a polyethylene glycol having 3 ppm total aldehyde content had less than 1% phenylephrine degradation; the sample made from a polyethylene glycol having 12 ppm total aldehyde concentration had less than 2% total phenylephrine degradation, and the sample made from a polyethylene glycol having 72 ppm total aldehyde concentration had nearly 4.5% total phenylephrine degradation. The rate of degradation for the sample having 72 ppm is problematic for commercial shelf life of a product.

**Claims**

1. An oral liquid pharmaceutical composition comprising:

(a) phenylephrine or a pharmaceutically acceptable salt thereof;

(b) substantially aldehyde-free polyethylene glycol, wherein the substantially aldehyde-free polyethylene glycol has less than 10 ppm total aldehyde content and maintains said level of aldehyde content for at least one year;
(c) a second active agent selected from an analgesic, a decongestant, an expectorant, an anti-tussive, an antipyretic, an anti-inflammatory agent, a cough suppressant and an antihistamine; and
(d) a buffering agent which maintains a pH below 5.4 in the composition.

2. The composition of claim 1 further comprising a flavor system wherein the flavor system includes non-aldehyde flavorants.

3. The composition of any one of claims 1 or 2, further comprising an antioxidant
wherein the antioxidant is propyl gallate.

4. An aqueous oral pharmaceutical composition according to claim 1 further comprising:

(i) an artificial sweetener;
(ii) up to w/v 45% glycerin; and
(iii) up to w/v 50% sorbitol.

5. The composition of claim 4 wherein the composition is an aqueous based solution.

6. The composition of any one of claims 4 to 5 wherein the buffering agent maintains a pH between about 2 and about 5 in the composition.

7. The composition of claim 4, further comprising a viscosity modifying agent.

8. The composition according to claim 7 wherein the viscosity modifying agent is selected from the group consisting of chitosan, microcrystalline cellulose, xanthan, HPMC, HPC, HEC, galactomannans and combinations thereof.

9. The composition of any one of claims 4 to 8, wherein the artificial sweetener is selected from the group consisting of sucralose, saccharine salts, cyclamates, acesulfame K, dipeptide based sweeteners, aspartame and mixtures thereof.

10. The composition of claim 9, wherein the artificial sweetener comprises sucralose.

**Patentansprüche**

1. Orale flüssige pharmazeutische Zusammensetzung, die umfasst:

(a) Phenylephrin oder ein pharmazeutisch verträgliches Salz davon;
(b) im Wesentlichen aldehydfreies Polyethylenglykol, wobei das im Wesentlichen aldehydfreie Polyethylenglykol einen Gesamtaldehydgehalt von weniger als 10 ppm hat und dieses Level an Aldehydgehalt mindestens ein Jahr beibehält;
(c) ein zweites aktives Mittel, ausgewählt aus einem Analgetikum, einem Dekongestionsmittel, einem Expectorans, einem Antihustenmittel, einem Antipyretikum, einem anti-inflammatorischen Mittel, einem Hustenhemmer und einem Antihistamin; und
(d) einem Puffermittel, das einen pH unterhalb 5,4 in der Zusammensetzung aufrecht erhält.

2. Zusammensetzung gemäß Anspruch 1, die weiterhin ein Aromasystem umfasst, wobei das Aromasystem nicht-aldehydische Aromamittel umfasst.

3. Zusammensetzung gemäß einem der Ansprüche 1 oder 2, die weiterhin ein Antioxidationsmittel umfasst, wobei das Antioxidationsmittel Propylgallat ist.

4. Wässrige orale pharmazeutische Zusammensetzung gemäß Anspruch 1, die weiterhin umfasst:

(i) einen künstlichen Süßstoff;
(ii) bis zu G/V 45% Glycerin; und

(iii) bis zu G/V 50% Sorbitol.

**5.** Zusammensetzung gemäß Anspruch 4, wobei die Zusammensetzung eine Lösung auf Wasserbasis ist.

**6.** Zusammensetzung gemäß einem der Ansprüche 4 bis 5, wobei das Puffermittel einen pH zwischen etwa 2 und etwa 5 in der Zusammensetzung aufrecht erhält.

**7.** Zusammensetzung gemäß Anspruch 4, die weiterhin ein Mittel zur Modifizierung der Viskosität umfasst.

**8.** Zusammensetzung gemäß Anspruch 7, wobei das Mittel zur Modifizierung der Viskosität ausgewählt ist aus der Gruppe, bestehend aus Chitosan, mikrokristalliner Cellulose, Xanthan, HPMC, HPC, HEC, Galactomannanen und Kombinationen davon.

**9.** Zusammensetzung gemäß einem der Ansprüche 4 bis 8, wobei der künstliche Süßstoff ausgewählt ist aus der Gruppe, bestehend aus Sucralose, Saccharinsalzen, Cyclamaten, Acesulfam K, Dipeptid-basierten Süßstoffen, Aspartam und Mischungen davon.

**10.** Zusammensetzung gemäß Anspruch 9, wobei der künstliche Süßstoff Sucralose umfasst.

**Revendications**

**1.** Composition pharmaceutique liquide orale comprenant :

(a) de la phényléphrine ou un sel pharmaceutiquement acceptable de celle-ci ;
(b) du polyéthylène glycol sensiblement exempt d'aldéhyde, le polyéthylène glycol sensiblement exempt d'aldéhyde ayant une teneur totale en aldéhyde inférieure à 10 ppm et conservant ledit niveau de teneur en aldéhyde pendant au moins un an ;
(c) un second agent actif sélectionné parmi un analgésique, un décongestionnant, un expectorant, un antitussif, un antipyrétique, un agent anti-inflammatoire, un suppresseur de toux et un antihistaminique ; et
(d) un agent tampon qui maintient un pH inférieur à 5,4 dans la composition.

**2.** Composition selon la revendication 1, comprenant en outre un système d'arôme, le système d'arôme incluant des aromatisants non-aldéhydiques.

**3.** Composition selon l'une quelconque des revendications 1 ou 2, comprenant en outre un antioxydant, l'antioxydant étant le gallate de propyle.

**4.** Composition pharmaceutique orale aqueuse selon la revendication 1, comprenant en outre :

(i) un édulcorant artificiel ;
(ii) jusqu'à 45 % en pds/v de glycérine ; et
(iii) jusqu'à 50 % en pds/v de sorbitol.

**5.** Composition selon la revendication 4, la composition étant une solution à base aqueuse.

**6.** Composition selon l'une quelconque des revendications 4 à 5, dans laquelle l'agent tampon maintient un pH entre environ 2 et environ 5 dans la composition.

**7.** Composition selon la revendication 4, comprenant en outre un agent modificateur de viscosité.

**8.** Composition selon la revendication 7, dans laquelle l'agent modificateur de viscosité est sélectionné dans le groupe consistant en le chitosane, la cellulose microcristalline, le xanthane, l'HPMC, l'HPC, l'HEC, les galactomannanes et les combinaisons de ceux-ci.

**9.** Composition selon l'une quelconque des revendications 4 à 8, dans laquelle l'édulcorant artificiel est sélectionné dans le groupe consistant en le sucralose, les sels de saccharine, les cyclamates, l'acésulfame K, les édulcorants à base de dipeptide, l'aspartame et les mélanges de ceux-ci.

**10.** Composition selon la revendication 9, dans laquelle l'édulcorant artificiel comprend du sucralose.

**Figure 1**

Effect of Various PEG's on PE Stability Liquid Products

**EP 2 351 554 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60774634 B